Europäisches Patentamt

European Patent Office

Office européen des brevets

(11) Publication number: **0 158 964**
**B1**

(12) **EUROPEAN PATENT SPECIFICATION**

(45) Date of publication of patent specification: **14.11.90**

(21) Application number: **85104308.3**

(22) Date of filing: **10.04.85**

(51) Int. Cl.⁵: **G 01 N 33/52,** G 01 N 33/72, C 12 Q 1/28, C 12 Q 1/54

(54) **Tester for detecting a substance in a body fluid.**

(30) Priority: **20.04.84 JP 79915/84**

(43) Date of publication of application:
**23.10.85 Bulletin 85/43**

(45) Publication of the grant of the patent:
**14.11.90 Bulletin 90/46**

(84) Designated Contracting States:
**BE DE FR IT**

(56) References cited:
**EP-A-0 016 962**
**EP-A-0 027 236**
**EP-A-0 037 056**
**EP-A-0 043 469**
**EP-A-0 103 958**
**DE-A-2 118 455**
**FR-A-2 247 730**
**GB-A-1 105 927**

(73) Proprietor: **TERUMO KABUSHIKI KAISHA trading as TERUMO CORPORATION**
**44-1, 2-chome, Hatagaya Shibuya-Ku**
**Tokyo 151 (JP)**

(72) Inventor: **Kaminagayoshi, Satoshi**
**24-19, Senbonkita 2-chome Nishinari-ku**
**Osaka-shi Osaka (JP)**

(74) Representative: **Henkel, Feiler, Hänzel & Partner**
**Möhlstrasse 37**
**D-8000 München 80 (DE)**

Courier Press, Leamington Spa, England.

## Description

### Field of the invention

This invention relates to a tester for detecting a substance in a body fluid designated to prevent reducing substances possibly present in a given specimen under test from exerting their effect upon a test piece for determining a hydroxyperoxide or hydrogen peroxide or some other hydroperoxide produced in the previous reaction by utilizing the peroxidase reaction or a peroxidase-like reaction, a test piece for detecting peroxidase or a substance acting like peroxidase, or a test piece for effecting measurement by utilizing the diazo coupling reaction. More particularly this invention relates to an improved tester for use in the detection of glucose or bilirubin in urine or blood, the detection of blood in urine, feces or other body fluid, or the detection of a nitrite of microorganic origin in urine.

### Description of prior art

Recently, quick diagnostic agents are gaining remarkably in significance. Those quick diagnostic agents which are based on absorbent carriers are important. Such an agent is prepared by impregnating an absorbent carrier, which is a slip of paper in most cases, with a chemical reagent necessary for the reaction utilized for the detection, and the agent is immersed in a given body fluid. The agent assumes a color reaction in the presence of the substance being detected. Quick diasgnostic agents which have become extremely important for medical diagnosis are those to be used for the detection of glucose or bilirubin in urine and blood, for the detection of occult blood in urine, feces and a body fluid, and for detection of a nitrite of microorganic origin in urine.

This test is based on the following principles.

### (1) Case concerning formation of hydroperoxide or hydrogen peroxide

This case applies to specimens containing glucose, uric acid, cholesterol, triglyceride and free fatty acids. The principle in this case will be described with reference to glucose taken as an example.

In the detection of glucose in body fluid, the glucose is oxidized by glucose oxidase (GOD) into gluconic acid. During this reaction, the oxygen in the air is reduced to hydrogen peroxide. This hydrogen peroxide, in the presence of peroxidase or a substance acting like peroxidase, causes oxidation and coloration of an oxidation indicator. The density of the color so generated serves as the criterion for the amount of glucose present.

### (2) Case concerning peroxidase or a substance acting like peroxidase

This case applies to specimens containing hemoglobin (occult blood) and myoglobin (renal disorder). The principle in this case will be described with reference to hemoglobin taken as an example. In the detection of occult blood, by the peroxidase-like activity of hemoglobin, the peroxide contained in advance in the test piece generates oxygen to color the oxidation indicator.

### (3) Case concerning diazo coupling reaction

This case applies to specimens containing nitrite (bacterial urine) and bilirubin (due to cholecystitis). The principle in this case resides in the fact that the reagent is colored by being diazotized with the aforementioned substance present in the specimen.

Various agents for rapid diagnosis such as are described above have been known in the art.

Various agent based on the principles described in (1) and (2) above are disclosed in Japanese Patent Laid-Open No. SHO 48(1973)-1120, Japanese Patent Publication Nos. SHO 58(1973)-3679, SHO 56(1981)-43238 and SHO 57(1982)-53539, and Japanese Patent Laid-Open No. SHO 53(1978)-16692, for example. Various agents based on the principle described in (3) above are disclosed in US—A—3,415,717, 3,547,780 and 3,718,543, Japanese Patent Laid-Open No. SHO 58(1983)-134067, Japanese Patent Publication Nos. SHO 48(1973)-1792, SHO 49(1974)-27719, SHO 53(1978)-27964, SHO 53(1978)-43431, SHO 53(1978)-27964, SHO 54(1979)-13508 and SHO 55(1980)-26426.

Recent processed foodstuffs and refreshing beverages contain ascorbic acid in large amounts as vitamin C and generous ingestion of vitamin preparations for the preservation of health are in vogue. Thus, such vitamins are richly contained in blood and excess water-soluble vitamins are excreted from the body in urine. When the aforementioned diagnostic agent is used in the test of urine or blood, therefore, there is entailed the problem that the strong reducing power exhibited by the ascorbic acid present in the urine or blood will interfere with the diazo coupling reaction and impede ample coloration. This disadvantage is suffered by the test piece which utilizes the diazo coupling reaction, one form of oxidation.

The adverse effect which the reducing substances such as ascorbic acid present in specimens exert upon the test results is pointed out in the aforementioned patent publications regarding the agents of the principles described in (1) and (2) above. The adverse effect with respect to the agents of the principle described in (3) above is not dealt with in any of the aforementioned patent publications. EP—A—0 037 056 discloses a test piece for the detection of redox reactions in which the tester contains the oxidizing agent (for ascorbic acid, for example) and the indicator in the same layer.

However, all the other reagents except the iodate are impregnated with one test paper in the first step and the iodate is impregnated as the second step, so both are merely impregnated with one test paper.

EP—A—0 103 958 discloses that "in a solid support 'dip-stick' immunoassay wherein a peroxidase catalyses the formation of a dye, ascorbate interference with the assay is reduced or eliminated by impregnating the support with peroxidase". Further, iodate and periodate are used to oxidate the interfering ascorbate.

GB—A—1 105 927 discloses a diagnostic composition for detection of a specific chemical substance in fluids containing interfering substances. For the interfering substances, the diagnostic composition contains a trapping agent capable of inactivating or removing reducing substances which may be present in the fluid being tested and tending to interfere with the detection of said specific chemical substance. GB—A—1,105,927 does not disclose $HIO_4$ or $MIO_4$ as an oxidizing agent nor does it disclose or suggest or arrange the chromogenic substance and the trapping agent in different layers of the diagnostic composition.

FR—A—2,247,730 discloses a test piece comprising a fluid permeable film on a test piece which comprises a substrate containing an indicator as that of the present invention. But in this disclosure an absorbent material treated with the indicator composition forms one single layer, over which a layer of non-treated absorbent material is provided. That is, FR—A—2,247,730 merely proposes and discloses to pile up two layers, and it does not teach to incorporate other agents on the indicating agent layer.

An object of this invention is to provide a test device designed to prevent reducing substances possibly present in a given specimen under test from exerting their effect upon a test paper for determining a hydroperoxide or hydrogen peroxide or some other hydroperoxide produced in the previous reaction by utilizing the peroxidase reaction or a peroxidase-like reaction, a test paper for detecting peroxidase or a substance acting like peroxidase, or a test piece for effecting measurement by utilizing the diazo coupling reaction.

## Summary of the invention

The object of this invention described above is accomplished by a tester for detecting a detectable substance in a sample of body fluid comprising, a stick, a test piece comprising a substrate secured to said stick and said substrate containing an indicator including (A) a diazotizing agent or (B) a coloring agent and one member selected from the group containing (a) a peroxidase and a glucose oxidase and (b) a hydroperoxidase or hydrogen peroxide, a fluid permeable film comprising membranous substrate covering said test piece; which is characterized in that said film containing an adhesive agent and an oxidizing agent selected from $NaIO_4$ and $HIO_4$ in an amount from 0.9 to 15 mmol/m², whereby the sample of body fluid, when applied to said film, passes through said film and reacts with the oxidizing agent before reaching the test piece; and said film being transparent or semi-transparent, at least, when contacted with said sample of body fluid, whereby color change in the test piece can be observed.

Since the substance in a specimen to be measured is brought into contact with the oxidizer film before it comes into contact with the indicator, the substance in a specimen coming into contact with the indicator is not appreciably affected by the reducing substances.

## Brief description of the drawings

Figure 1 is a cross-sectional view illustrating a typical tester as one embodiment of this invention, and

Figure 2 is a cross-sectional view illustrating a typical tester as another embodiment of this invention.

## Description of the preferred embodiment

The tester according with this invention has an oxidizer film deposited on a test piece for the purpose of preventing reducing substances possibly present in a given specimen under test from manifesting their effect upon a test piece for determining a hydroperoxide or hydrogen peroxide or some other hydroperoxide produced in a previous reaction by utilizing the peroxidase reaction or a peroxidase-like reaction, a test piece for detecting peroxidase or a substance acting like peroxidase, or a test piece for effecting measurement by utilizing the diazo coupling reaction.

The aforementioned oxidizer film is transparent or semi-transparent (at least when the film is wet with the specimen under test) and, therefore, permits any change of color of the water-absorbing carrier to be seen therethrough. It comprises a membranous substrate having a reticular structure or containing a multiplicity of pinholes and an oxidizing agent deposited by impregnation or coating on the membranous substrate. The membranous substrate may be formed of non-waven fabric or porous membrane of plastic substance, glass fibers, translucent membrane, or filter paper.

The amount of the oxidizing agent in the aforementioned oxidizer film is desired to fall in the range of 0.9 to 15 mmoles per unit area (m²) of the test piece. If this amount is less than 0.9 mmol/m², the supply of the oxidizing agent is not sufficient for ample oxidization of the reducing substances in the specimen. If it exceeds 15 mmols/m², the test piece is caused to assume color (indicating positive test) even when the specimen contains no reducing substances.

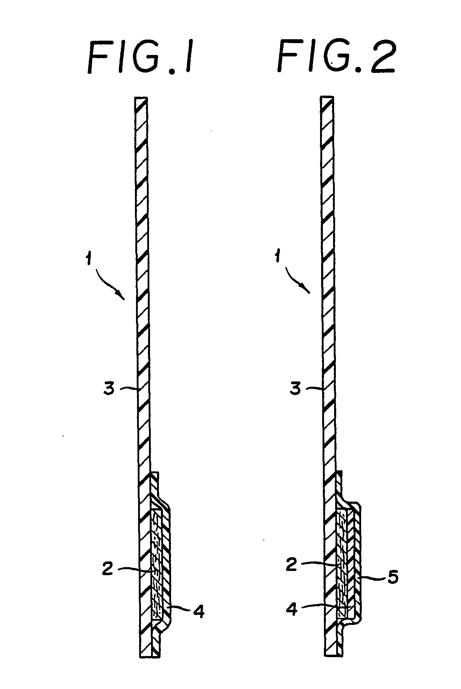

Typical constructions of a tester 1 according to this invention are represented in lateral elevations in Figures 1 and 2. With reference to Figure 1, a test piece 2 is held fast on a stick 3 by means of an oxidizer film 4. With reference to Figure 2, a test paper 2 and an oxidizer film 4 are superposed and are held fast on a stick 3 by means of a mesh 5 adapted exclusively for fixing the test piece in place. The body fluid such as urine or blood which is given as a specimen permeates one oxidizer film 4, wherein the reducing substance in the specimen is oxidized and then delivered to the test piece. By the specimen, the test piece is caused to assume color without being affected by the reducing substance in the specimen. As the result, the specimen is given a normal test.

The oxidizing agent to be incorporating for the oxidation of the reducing substance present in the specimen is $NaIO_4$ and $HIO_4$.

Specifically, although the amount of the oxidizing agent is affected by the amount of the oxidation indicator contained in the aforementioned tester and the amount of the reducing substance present in the specimen, the oxidizing agent is desired to be contained in the oxidizer film in an amount of 0.9 to 15 mmoles per unit area ($m^2$) of the test piece. The test piece enjoys greater stability when the oxidizing agent is incorporated in the film superposed on the test piece than when it is incorporated directly in the test piece. The oxidation indicator intended to assume color by undergoing gradual oxidation participates in the reaction which forms an oxidative condensate as the source for the measurement of the oxygen produced by the peroxidase or by the peroxidase-like reaction (phenol type and 4-aminoantipyrine), the reaction which forms acetyl acetone and a condensate by the oxidation of methanol into formaldehyde, the reaction which forms iodine by the oxidation of an iodide such as potassium iodide, and the diazotization.

The oxidative indicator which is oxidized and enabled to color serves to detect the oxygen produced by the peroxidase or peroxidase-like reaction and, in that capacity, participates in the reaction producing oxidized condensates (phenol, with 4-aminoantipyrine), the reaction causing methanol to be oxidized into formaldehyde and then combined with acetylacetone to produce a condensate, and the reaction causing an iodide such as potassium iodide to be oxidized to produce iodine.

As coloring agents, benzidine type compounds, heterocyclic azines, phenols and guaiacum resin components are known to the art. Typical examples include o-tolidine, m-tolidine, bendizine, 3,3',5,5'-tetramethyl benzidine, o-methyl benzidine, 2,7-diaminofluorene, mixtures containing these compounds in various proportions, various substituted phenylene diamines such as 4,4'-diaminodiphenyl, o-phenylene diamine, m-phenylene diamine, p-phenylene diamine, 2,3-tolylene diamine, 2,4-tolylene diamine, 2,5-tolylene diamine and 2,6-tolylene diamine, pyrogallic acid, gallic acid, phenols such as fluoroglucinol, hydroquinone, and leucoindophenol, guaiacol, pyridine derivatives, substituted azines and leuco-malachite green. The oxidative indicator is used generally in an amount of 2 to 250 mmol/l, preferably in an amount of 9 to 50 mmol/l.

Typical diazo compounds are p-diazobenzene sulfonic acid, 2,6-dichlorobenzene diazonium tetrafluoroborate, 2-trifluoromethylbenzene diazonium tetrafluorobarate and 2,4-dichlorobenzene diazonium tetrafluoroborate.

Examples of the hydroperoxide to be used in this invention include 2,5-dimethylhexane-2,5-dihydroperoxide, cumene hydroperoxide, 2,5-dimethylhexanone-2,5-dihydroperoxide, diisopropylbenzene hydroperoxide, t-butyl hydroperoxide, p-methane hydroperoxide and 4-methylphenylisopropyl hydroperoxide. Among other hydroperoxides enumerted above, 2,5-dimethylcyclohexanone-2,5-dihydroperoxide and cumene hydroperoxide are desirable hydroperoxides having relatively low decomposition ratios.

The tester of the present invention is used in the form of a test piece attached to a plastic stick and the like singly or plurally. The test piece attached to the stick may optionally incorporate therein a buffer or a lubricant. The buffer serves to retain the pH value of the fluid in which the test piece is immersed in the range of 4 to 8, preferably 5 to 7. Examples of the buffer are citric acid-sodium citrate, tartaric acid-sodium tartrate, malic acid-borax, potassium hydrogen phthalate-dipotassium phthalate and sodium hydrogen succinate-disodium succinate.

The wetting agent serves to enable the body fluid in which the test piece is immersed to uniformly wet the test piece. Examples of the wetting agent are surface active agents including alkyl sulfonic acid salts such as sodium lauryl sulfate, sodium dodecyl sulfate and sodium tetradecyl sulfate, alkylbenzene sulfonates such as sodium dodecylbenzene sulfonate and dialkylsulfosuccinates such as sodium dioctyl-sulfosuccinate, sodium diheptylsulfosuccinate and cetyl pyridinium chloride.

Further, the aforementioned tester may incorporate therein a chemical agent capable of enhancing the peroxidase activity in hemoglobin. Typical enhancers usable for this purpose include quinoline and derivatives thereof, such as quinine, cinchonine, o-methoxyquinoline, quinalidine, 8-amino-6-methoxy quinoline, 2-quinolinol, isoquinoline, benzo(f)quinoline and 3-amino-quinoline. In the presence of such as enhancer, the oxidation reaction is generally accelerated and the oxidized chromagen has its color-generating strength enhanced. Consequently, the test piece acquires increased sensitivity.

It may incorporate therein an adhesive agent for the purpose of preventing exudation of the agent from the test piece. Typical adhesive agents are polymers such as polyvinyl alcohol, polyvinyl pyrrolidone, polyethylene glycol, polyacrylates, polyacrylamide, poly(hydroxyethyl methacrylate), poly(hydroxyethyl acrylate), carboxymethyl cellulose, gelatin, gum arabic and methylcellulose.

A testing agent is used in the form a solution. Examples of the solvent to be used in the preparation of such a solution are benzene, toluene, xylene, methanol, ethanol, isopropanol, acetone, methylethyl ketone, chloroform, carbon tetrachloride and water. Otherwise, the tester is used in the form of a test piece which is obtained by impregnating a substrate with the aforementioned solution and drying the wet substrate. The test piece is manufactured by an ordinary method.

As the substrate for the test piece, filter paper and other similar forms of paper, non-woven fabric made

4

of glass fibers or plastic material and water absorbing plastic sheet are available. The substrate so selected fits the preparation of the test piece when it fulfils the requirement that it should neither react upon nor dissolve in the impregnating solution and should possess an absorbing property. Paper is a preferred example.

The testing agent of the present invention is used for the various medical diagnosis.

The tester is useful (A) for the detection of a hydroperoxide or hydrogen peroxide or other hydroperoxide produced in a preceding reaction through the agency of a peroxidase or a peroxidase-like reaction, (B) for the detection of a peroxidase or a peroxidase-acting substance, and (C) for the detection using diazo coupling reaction. The substances which can be thus detected by the tester of this invention include glucose, uric acid, hemoglobin, myoglobin, nitrites, bilirubin in urine, and glucose, urinc acid, cholesterol, triglyceride, free fatty acid, hemoglobin and bilirubin in blood for example.

The tester of the present invention can be used in any desired shape such as a stick or a test piece. Now, the operation of the test of this invention will be described below with reference to an embodiment wherein the tester is constructed in the shape of a stick as illustrated in Figure 1 and the specimen contains ascorbic acid as a reducing substance.

The tester of the present invention has the oxidizer film for elimination of the adverse effect of reducing substance present in the specimen deposited on a test piece which has to do with hydroperoxide or with a reaction giving rise to hydroperoxide, a test piece which is intended for determination of peroxidase or a substance action like peroxidase, or a test piece which is used for determination by virtue of the diazo coupling reaction.

Owing to the construction, therefore, the specimen such as urine or blood first permeates the aforementioned oxidizer film. The reducing substance (such as ascorbic acid) present in the specimen is oxidized by the aforementioned oxidizing agent and then brought into contact with the test piece. Thus, the test piece is colored by this contact. Thus, even when the specimen happens to contain any reducing substance, the reducing substance is not suffered to exert any adverse effect upon the results of determination.

The reducing substance (such as ascorbic acid) present in the specimen and the oxidizing agent in the oxidizer film have their effect to bear upon the test results through the following actions.

(1) Reaction relating to hydroperoxide or participating in the formation of hydroperoxide

(1—1) In the presence of ascorbic acid, the nascent [0] produced from $H_2O_2$ by the peroxidase is spent for the oxidation of ascorbic acid and, as the result, the subsequent color reaction is impeded.

(1—2) When the aforementioned oxidizing agent is contained in advance in the test piece, the oxidizing agent and the ascorbic acid undergo an oxidation-reduction reaction before the occurrence of the nascent [0] and, therefore, the ascorbic acid is prevented from interfering with the reaction.

(2) Peroxidase or substance acting like peroxidase Same as (1—1) and (1—2).

(3) Diazo coupling reaction.

The diazo coupling reaction is an oxidative reaction. Thus, the reaction is obstructed by the presence of ascorbic acid. When the oxidizing agent is present, this obstruction of the reaction cannot occur because the oxidation-reduction reaction between the oxidizing agent and the ascorbic acid preceeds faster.

Now, the present invention will be described more specifically below with reference to working examples.

Before the relevant experiments, the tester constructed as shown in Figures 1 and 2 were prepared. First, a dope for the oxidizer film was prepared by dissolving $NaIO_4$ as an oxidizing agent in a concentration of 1 mg/ml in an aqueous 2.5% methyl cellulose solution. A nylon net was immersed in the solution and dried to form an oxidizer film. For the convenience of reference, this oxidizer film will be referred to hereinafter as "ascorbic acid removing film." This film was deposited each on a stick in a manner embracing a test piece therein as illustrated in Figures 1 and 2. This ascorbic acid removing film was found to contain the oxidizing agent in a concentration of 2 m.mols per $m^2$ of the area of the test piece.

Example 1

Test for glucose

The ascorbic acid removing film obtained as described above was superposed on test papers for glucose to produce tester constructed as illustrated in Figures 1 and 2. These testers were immersed in urine containing no glucose and urine containing glucose in a concentration of 150 mg/dl. Both urine contained ascorbic acid in a concentration of 50 mg/dl. Tester lacking an ascorbic acid removing film was similarly used in the experiment. The results were as shown in Table 1.

TABLE 1

| Ascorbic acid removing film | Film of | | No film used |
|---|---|---|---|
| | Fig. 1 | Fig. 2 | |
| Urine (no glucose) | − | − | − |
| Urine (containing glucose in 150 mg/dl and ascorbic acid in 50 mg/dl | + | + | − |

Example 2

Test for occult blood

The ascorbic acid removing film obtained as described above was superposed on test pieces for occult blood to produce tester constructed as shown in Figures 1 and 2. These testers were immersed in urine containing no hemoglobin and urine containing hemoglobin in a concentration of 15 mg/dl. Both urines contained ascorbic acid in a concentration of 50 mg/dl. Tester lacking an ascorbic acid removing film was similarly used in the experiment. The results were as shown in Table 2.

TABLE 2

| Ascorbic acid removing film | Film of | | No film used |
|---|---|---|---|
| | Fig. 1 | Fig. 2 | |
| Urine (no hemoglobin) | − | − | − |
| Urine (containing hemoglobin in 15 mg/dl and ascorbic acid in 50 mg/dl | + | + | − |

Example 3

Test for bilirubin

The ascorbic acid removing film obtained as described above was superposed on test pieces for bilirubin to produce tester constructed as shown in Figures 1 and 2. These testers were immersed in urine containing no bilirubin and urine containing bilirubin in a concentration of 0.5 mg/dl. Both urines contained ascorbic acid in a concentration of 50 mg/dl. Tester lacking an ascorbic acid removing film was similarly used in the experiment. The results were as shown in Table 3.

TABLE 3

| Ascorbic acid removing film | Film of | | No film used |
|---|---|---|---|
| | Fig. 1 | Fig. 2 | |
| Urine (no bilirubin) | − | − | − |
| Urine (containing bilirubin in 0.5 mg/dl and ascorbic acid in 50 mg/dl | + | + | − |

Example 4

Test for nitrite

The ascorbic acid removing film obtained as described above was superposed on test pieces for nitrite to produce tester constructed as shown in Figures 1 and 2. These testers were immersed in urine containing no nitrite and urine containing nitrite (ion) in a concentration of 0.1 mg/dl. Both urines contained ascorbic acid in a concentration of 50 mg/dl. Tester lacking an ascorbic acid removing film was similarly used in the experiment. The results were as shown in Table 4.

TABLE 4

| Ascorbic acid removing film | Film of | | No film used |
|---|---|---|---|
| | Fig. 1 | Fig. 2 | |
| Urine (no nitrite) | − | − | − |
| Urine (containing nitrite in 0.5 mg/dl and ascorbic acid in 50 mg/dl | + | + | − |

Example 5

A solution for an oxidizer film was prepared by dissolving NaIO₄ as an oxidizing agent in a concentration of 4.5 mg/ml in an aqueous 2.5% methyl cellulose solution. A net was immersed in the solution and dried to produce an oxidizer film. The oxidizer film was superposed on test piece for glucose as illustrated in Figures 1 and 2. The oxidizer film was found to contain the oxidizing agent in a concentration of 21 m.mols per m² of the area of the test piece.

The tester obtained as described above were immersed in urine containing no glucose and urine containing glucose in a concentration of 150 mg/dl. Both urines contained ascorbic acid in a concentration of 50 mg/dl. The results were as shown in Table 5.

It is noted from Table 5 that an excess of the oxidizing agent gives an undesirable result.

TABLE 5

| Ascorbic acid removing film | Film of | | No film used |
|---|---|---|---|
| | Fig. 1 | Fig. 2 | |
| Urine (no glucose) | + | + | − |
| Urine (containing glucose in 150 mg/dl and ascorbic acid in 50 mg/dl | + | + | − |

The test pieces used in Examples 1—5 were prepared as described below. In the preparation of each test piece, a paper substrate was first impregnated with Solution A and dried. It was then subjected to the same procedure with respect to Solution B and solution C each in the order mentioned.

    (a)  Test piece for glucose
         Solution A
            Citrate buffer               100 ml
            Glucose oxidase         300 mg
            Peroxidase                50 mg
         Solution B
            Ortho-tolidine           2.5 g
            Acetone                 100 ml

    (b)  Test piece of nitrite
         Solution A
            Arsanilic acid           0.8 g
            Methanol              100 ml
         Solution B
            Naphthylethylene diamine 2HCl 0.9 g
            Methanol              100 ml

(c) Test piece for bilirubin
Solution A

| | |
|---|---|
| p-Diazobenzene sulfonic acid | 300 mg |
| Metaphosphoric acid | 15 g |
| Distilled water | 100 ml |

Solution B

| | |
|---|---|
| Cetylpyridinium chloride | 3.5 g |
| Ethanol | 100 ml |

(d) Test piece for occult blood
Solution A

| | |
|---|---|
| 2,5-Dimethylhexane-2,5-dihydroperoxide | 1.2 g |
| Ethanol | 100 ml |

Solution B

| | |
|---|---|
| Sodium citrate | 9 g |
| Citric acid | 1 g |
| Saponin | 100 mg |
| Distilled water | 100 ml |

Solution C

| | |
|---|---|
| Ortho-tolidine | 1.2 g |
| Benzene | 100 ml |

As described above, in the tester of the present invention having an oxidizer film deposited on a test piece for determining a hydroperoxide or hydrogen peroxide or some other hydroperoxide produced in a previous reaction by utilizing the peroxidase reaction or a peroxidase-like reaction, a test piece for detecting peroxidase or a substance acting like peroxidase, or a test piece for effecting determination by utilizing the diazo coupling reaction, the testing agent comprises an oxidation indicator selected from the group consisting of the aforementioned hydroperoxide, substance acting like peroxidase, and diazotizable substance and, therefore, allowed to develop color in consequence of the oxidation in the reaction of the substance so selected and an effective amount of a specific oxidizing agent. When the specimen such as urine or blood given to be tested happens to contain a reducing substance such as ascorbic acid or gentisic acid, the peroxidase reaction, the peroxidase-like reaction or the diazo coupling reaction is not obstructed by the reducing substance because the reducing substance is oxidized by the aforementioned oxidizing agent. Even when the aforementioned oxidizing agent is contained in advance in the oxidizer film, there is no possibility of the peroxidase reaction, peroxidase-like reaction, or diazo coupling reaction being induced by the oxidizing agent. The effect of the device has been found to be especially high for the oxidizing agent of the invention namely for periodic acid or a salt thereof. The effect of the invention is especially excellent when the tester uses an oxidizer film containing this oxidizing agent in a concentration of 0.9 to 15 mmol/$m^2$ of the area of test piece. Since the aforementioned oxidizing agent is contained in the oxidizer film and, therefore, is separated from the test piece, the tester enjoys greater stability to resist the effect of aging.

**Claims**

1. A tester for detecting a detectable substance in a sample of body fluid comprising, a stick, a test piece comprising a substrate secured to said stick and said substrate containing an indicator including (A) a diazotizing agent or (B) a coloring agent and one member selected from the group containing (a) a peroxidase and a glucose oxidase and (b) a hydroperoxidase or hydrogen peroxide, a fluid permeable film comprising membranous substrate covering said test piece; characterized in that said film containing an adhesive agent and an oxidizing agent selected from $NaIO_4$ and $HIO_4$ in an amount from 0.9 to 15 mmol/$m^2$, whereby the sample of body fluid, when applied to said film, passes through said film and reacts with the oxidizing agent before reaching the test piece; and said film being transparent or semi-transparent, at least, when contacted with said sample of body fluid, whereby color change in the test piece can be observed.

2. A tester according to claim 1, wherein said substrate is selected from paper, non-woven fabric of glass fibers or plastic material and water-absorbing plastic sheet.

3. A tester according to claim 1 and/or 2, wherein said membrane substrate is selected from papers, non-woven fabric of glass fiber or plastic material and water-absorbing plastic sheet.

4. A tester according to any of claims 1 to 3, wherein said tester is intended for glucose test.

5. A tester according to any of claims 1 to 3, wherein said tester is intended for occult blood test.

6. A tester according to any of claims 1 to 3, wherein said tester is intended for bilirubin test.

7. A tester according to any of claims 1 to 3, wherein said tester is intended for nitrite test.

8. A tester according to any of claims 1 to 7, wherein surface of said film is further covered with a mesh for fixing and fixed to a stick.

9. The tester according to any of claims 1 to 8, wherein said adhesive agent is at least one member selected from polyvinyl alcohol, polyvinyl pyrrolidone, polyethylene glycol, polyacrylates, polyacrylamide, poly(hydroxyethyl methacrylate), poly(hydroxyethyl acrylate), carboxymethyl cellulose, gelatin and gum arabic.

10. A tester according to any of claims 1 to 8, wherein said adhesive agent is methylcellulose.

**Patentansprüche**

1. Testbesteck zum Nachweis einer nachweisbaren Substanz in einer Probe einer Körperflüssigkeit, umfassend ein Stäbchen, ein Teststück mit einem an dem Stäbchen befestigten Substrat, welches einen Indikator mit (A) einem Diazotierungsmittel oder (B) einem Färbemittel und einem Bestandteil aus der (a) eine Peroxidase und eine Glucoseoxidase und (b) eine Hydroperoxidase oder Wasserstoffperoxid entumfassenden Gruppe enthält, und einen das Teststück bedeckenden, fluidumdurchlässigen Film, umfassend ein membranartiges Substrat, dadurch gekennzeichnet, daß der Film ein Klebemittel und ein aus NaIO$_4$ und HIO$_4$ ausgewähltes Oxidationsmittel in einer Menge von 0,9 bis 15 mMol/m$^2$ enthält, wobei die Probe der Körperflüssigkeit nach Applikation auf den Film durch den Film hindurchtritt und mit dem Oxidationsmittel reagiert, bevor sie das Teststück erreicht und daß der Film zumindest beim Inkontaktkommen mit der Probe der Körperflüssigkeit durchsichtig oder halbdurchsichtig ist, um eine Farbänderung im Teststück feststellen zu können.

2. Testbesteck nach Anspruch 1, dadurch gekennzeichnet, daß das Substrat aus Papier, einem Gespinst oder Gewirk aus Glasfasern oder Kunststoffmaterial oder einer wasserabsorbierenden Kunststofflage ausgewählt ist.

3. Testbesteck nach Anspruch 1 und/oder 2, dadurch gekennzeichnet, daß das Membransubstrat aus Papiersorten, einem Gewirk oder Gespinst aus Glasfasern oder Kunststoffmaterial oder einer wasserabsorbierenden Kunststofflage ausgewählt ist.

4. Testbesteck nach einem der Ansprüche 1 bis 3, dadurch gekennzeichnet, daß es für einen Glucosetest bestimmt ist.

5. Testbesteck nach einem der Ansprüche 1 bis 3, dadurch gekennzeichnet, daß es für einen Okkultbluttest bestimmt ist.

6. Testbesteck nach einem der Ansprüche 1 bis 3, dadurch gekennzeichnet, daß es für einen Bilirubintest bestimmt ist.

7. Testbesteck nach einem der Ansprüche 1 bis 3, dadurch gekennzeichnet, daß es für einen Nitrittest bestimmt ist.

8. Testbesteck nach einem der Ansprüche 1 bis 7, dadurch gekennzeichnet, daß die Oberfläche des Films zusätzlich mit einer netzartigen Befestigung bedeckt und an einem Stäbchen befestigt ist.

9. Testbesteck nach einem der Ansprüche 1 bis 8, dadurch gekennzeichnet, daß das Klebemittel mindestens ein aus Polyvinylalkohol, Polyvinylpyrrolidon, Polyethylenglykol, Polyacrylaten, Polyacrylamid, Poly(hydroxyethylmethacrylat), Poly(hydroxyethylacrylat), Carboxymethylcellulose, Gelatine und Gummiarabikum ausgewählter Bestandteil ist.

10. Testbesteck nach einem der Ansprüche 1 bis 8, dadurch gekennzeichnet, daß das Klebemittel aus Methylcellulose besteht.

**Revendications**

1. Un dispositif d'essai pour détecter une substance détectable dans un échantillon d'humeur de l'organisme, comprenant: une baguette, une pièce d'essai comprenant un substrat fixé à ladite baguette et ledit substrat contenant un indicateur contenant (A) un agent diazotant ou (B) un agent colorant et un composant choisi parmi (a) une peroxydase et une glucose-oxydase et (b) une hydroperoxydase ou le peroxyde d'hydrogène, un film perméable aux liquides comprenant une membrane de substrat recouvrant ladite pièce d'essai; caractérisé en ce que ledit film contient un agent adhésif et un agent oxydant choisi parmi NaIO$_4$ et HIO$_4$ en quantité de 0,9 à 15 mmol/m$^2$, de sorte que l'échantillon d'humeur de l'organisme, lorsqu'il est appliqué audit film, passe à travers ledit film et réagit avec l'agent oxydant avant d'atteindre la pièce d'essai; et en ce que ledit film est transparent ou semi-transparent, au moins, lorsqu'il est mis en contact avec ledit échantillon d'humeur de l'organisme, ce qui permet d'observer un changement de couleur dans la pièce d'essai.

2. Un dispositif d'essai selon la revendication 1, dans lequel ledit substrat est choisi parmi le papier, une étoffe non tissée de fibres de verre ou de matière plastique et une feuille de matière plastique absorbant l'eau.

3. Un dispositif d'essai selon la revendication 1 et/ou la revendication 2, dans lequel ladite membrane de substrat est choisie parmi les papiers, les étoffes non tissées de fibres de verre ou de matière plastique et les feuilles de matière plastique absorbant l'eau.

4. Un dispositif d'essai selon l'une quelconque des revendications 1 à 3, dans lequel ledit dispositif d'essai est destiné au test du glucose.

5. Un dispositif d'essai selon l'une quelconque des revendications 1 à 3, dans lequel ledit dispositif d'essai est destiné au test du sang occulte.

6. Un dispositif d'essai selon l'une quelconque des revendications 1 à 3, dans lequel ledit dispositif d'essai est destiné au test de la bilirubine.

7. Un dispositif d'essai selon l'une quelconque des revendications 1 à 3, dans lequel ledit dispositif d'essai est destiné au test des nitrites.

8. Un dispositif d'essai selon l'une quelconque des revendications 1 à 7, dans lequel la surface dudit film est encore recouverte par une toile pour fixer et fixée à une baguette.

9. Le dispositif d'essai selon l'une quelconque des revendications 1 à 8, dans lequel ledit agent adhésif est au moins un composant choisi parmi l'alcool polyvinylique, la polyvinylpyrrolidone, le polyéthylènegly-col, les polyacrylates, le polyacrylamide, le polyméthacrylate d'hydroxyéthyle, le polyacrylate d'hydroxy-éthyle, la carboxyméthylcellulose, la gélatine et la gomme arabique.

10. Un dispositif d'essai selon l'une quelconque des revendications 1 à 8, dans lequel ledit agent adhésif est la méthylcellulose.

# FIG.1     FIG.2